(19) **Europäisches Patentamt**

**European Patent Office**

**Office européen des brevets**

(11) **EP 0 793 976 B1**

(12) **EUROPÄISCHE PATENTSCHRIFT**

(45) Veröffentlichungstag und Bekanntmachung des Hinweises auf die Patenterteilung:
**07.07.2004 Patentblatt 2004/28**

(51) Int Cl.⁷: **A61N 1/365**

(21) Anmeldenummer: **97250057.3**

(22) Anmeldetag: **04.03.1997**

(54) **Ratenadaptiver Herzschrittmacher**

Rate adaptive pacemaker

Stimulateur cardiaque à fréquence adaptable

(84) Benannte Vertragsstaaten:
**DE FR GB IT NL SE**

(30) Priorität: **04.03.1996 DE 19609382**

(43) Veröffentlichungstag der Anmeldung:
**10.09.1997 Patentblatt 1997/37**

(73) Patentinhaber: **BIOTRONIK Mess- und Therapiegeräte GmbH & Co Ingenieurbüro Berlin
12359 Berlin (DE)**

(72) Erfinder: **Lang, Martin, Dr.
91091 Grossenseebach (DE)**

(74) Vertreter: **Eisenführ, Speiser & Partner
Patentanwälte Rechtsanwälte
Spreepalais am Dom
Anna-Louisa-Karsch-Strasse 2
10178 Berlin (DE)**

(56) Entgegenhaltungen:
**EP-A- 0 576 114      EP-A- 0 616 819
DE-A- 4 111 505      US-A- 5 074 302**

**Beschreibung**

Die Erfindung betrifft einen ratenadaptiven Herzschrittmacher gemäß dem Oberbegriff des Anspruchs 1.

Es sind Herzschrittmacher bekannt, die die adaptive Herzrate in Abhängigkeit von der Belastung des Herzschrittmacherträgers einstellen.

In der DE-C-34 19 439 ist ein Herzschrittmacher beschrieben, der mit einem Temperaturfühler die Bluttemperatur des venösen Blutes im Herzen mißt und die adaptive Herzrate in Abhängigkeit von dem gemessenen Wert einstellt. Diesem Prinzip liegt die Erkenntnis zugrunde, daß die Bluttemperatur des Menschen bei Belastung ansteigt: Die Zuordnung der Bluttemperatur zu der physiologisch sinnvollen adaptiven Herzrate erfolgt dabei durch eine Kennlinie, die jedem Wert der Bluttemperatur einen Wert der adaptiven Herzrate zuordnet.

Nachteilig bei diesem vorbekannten Herzschrittmacher ist, daß der Zusammenhang von Bluttemperatur und physiologisch sinnvoller Herzrate in der Regel für jeden Menschen unterschiedlich ist, so daß der Herzschrittmacher für jeden Herzschrittmacherträger individuell kalibriert werden muß.

Daruberhinaus führt eine belastungsunabhängige Änderung der Bluttemperatur - beispielsweise durch Alterung des Temperaturfühlers oder durch eine Verschiebung des Temperaturfühlers im Körper des Herzschrittmacherträgers - ebenfalls zu einer Änderung der adaptiven Herzrate, was physiologisch nicht sinnvoll ist.

Es ist eine Vielzahl von Anordnungen zur Impedanzmessung im Bereich des Thorax oder im Herzen zur Gewinnung eines Impedanzsignals für ratanadaptive Herzschrittmacher bekannt, so daß die Technik der intrakardialen Impedanzmesung als solche dem Fachmann vertraut ist. Die meisten dieser Anordnuneg streben aber eine Aussage zum Atem- bzw. Minutenvolumen als Ausdruck der körperlichen Belastung des Patienten und als eigentlichem Ratensteuerparameter an.

Bekannt ist auch das sogenannte ResQ-Verfahren (Regional Effective Slope Quality) (SCHALDACH, Max: Electrotherapy of the Heart, 1.Aufl., Springer-Verlag, S.114ff.), bei dem der zeitliche Verlauf der intrakardialen Impedanz zur Bestimmung der physiologisch sinnvollen adaptiven Herzrate herangezogen wird.

Diesem Verfahren liegt die Erkenntnis zugrunde, daß die intrakardiale Impedanz in einem bestimmten Zeitfenster nach einem QRS-Komplex - der sogenannten "region of interest" (ROI) - eine besonders signifikante Abhängigkeit von der Belastung des Organismus aufweist.

Es wird deshalb die Steigung der Impedanzkurve in der ROI bestimmt und die Differenz zwischen der Steigung einer Ruhe- bzw. Referenzkurve und der Steigung der aktuell gemessenen Impedanzkurve (Belastungskurve) berechnet. In Abhängigkeit von dieser Differenz wird die adaptive Herzrate eingestellt. Die Zuordnung der berechneten Steigungsdifferenz zu der einzustellenden Herzrate erfolgt auch hier durch eine Kennlinie. Da dieser Zusammenhang in der Regel für verschiedene Menschen unterschiedlich ist, muß der Herzschrittmacher für jeden Träger individuell kalibriert werden, und die Kalibrierung ist bei wesentlich verändertem Gesundheitszustand und Belastungsvermögen oder veränderten Lebensumständen des Patienten zu wiederholen, wobei auch die Lage der ROI zu überprüfen ist.

Aus der EP 0 576 114 und der DE 41 11 505 sind jeweils ratenadaptive Herzschrittmacher bekannt, die die Stimulationsrate in Abhängigkeit des Integrals des zeitlichen Verlaufs der intrakardialen Impedanz einstellen.

Es ist deshalb insbesondere die Aufgabe der Erfindung, einen gattungsgemäßen Herzschrittmacher zu schaffen, der ohne patientenspezifischen Kalibrierungsvorgang auskommen kann und sich an veränderte Randbedingungen selbsttätig anpaßt.

Diese Aufgabe wird durch die in Anspruch 1 angegebenen Merkmale gelöst.

Die Erfindung schließt den Gedanken ein, den - aufgrund der Verknüpfungen über das autonome Nervensystem (ANS) die Gesamt-Belastungssituation (physische und psychische Belastung) eines Patienten ausgezeichnet reflektierenden - zeitlichen Verlauf der intrakardialen (speziell rechtsventrikulären) Impedanz in einer aussagekräftigen, keine patientenindividuelle Einstellung erfordernden Größe zur Ratenadaption zu nutzen.

Der erfindungsgemäße Herzschrittmacher wertet die intrakardiale Impedanz, insbesondere die unipolar gemessene rechtsventrikuläre Impedanz, in einem breiten Bereich aus, der die üblicherweise für einzelne Patienten eingestellten ROI-Bereiche einschließt. Er bestimmt in diesem eine Relation zwischen Ruhebzw. Referenz- und Belastungskurve, speziell anhand einer arithmetischen Verarbeitung des Zeitintegrals der Impedanz über den genannten Bereich.

Hierzu ist der Ausgang der entsprechenden Integratorstufe insbesondere mit einem Integralwertspeicher verbunden, in den jeweils ein in mindestens einem vorhergehenden Herzzyklus ermittelter Referenz-Integralwert gespeichert wird, und die Ratenbestimmungseinrichtung umfaßt eine mit dem Ausgang der Integratorstufe und dem Integralwertspeicher verbundene Arithmetikeinheit zur Berechnung einer sekundären Impedanzgröße aus der jeweiligen primären Impedanzgröße und dem Referenz-Integralwert gemäß einer vorgegebenen arithmetischen Beziehung. Die Arithmetikeinheit ist in einer vorteilhaft einfachen Ausführung eine Subtraktionsstufe zur Bildung des Differenzwertes zwischen der primären Impedanzgröße und dem Referenz-Integralwert - es kann aber auch eine andere arithmetische Verarbeitung oder ggfs. auch eine mehrstufige Schwellwert-Diskrimination erfolgen.

Die Festlegung der Zeitbereichs- bzw. Integrationsgrenzen bedarf keiner patientenindividuellen Programmierung nach der Implantation, sondern diese können insbesondere bei der Herstellung des Schrittmachers in einem (mindestens mittelbar) mit einem Steuereingang der Integratorstufe verbundenen Festwertspeicher gespeichert werden. Die Grenzen des vorbestimmten Abschnitts werden im Ergebnis von Untersuchungen des für die Ratenadaption relevanten Bereichs des zeitlichen Verlaufs der Impedanz an einer Patientenpopulation ermittelt.

Die genannte Ruhe- bzw. Referenzkurve wird erfindungsgemäß "mitgeführt", d.h. aus über eine vorbestimmte Zeitspanne von einigen (beispielsweise drei) Minuten gewonnenen Impedanzwerten gemittelt, wobei entweder eine gleitende Mittelung oder jeweils eine Mittelung für aufeinanderfolgende getrennte Zeiträume vorgenommen werden kann. Dadurch wird eine schnelle Adaption an sich verändernde Randbedingungen - wie Stimulationsparameter, Medikation oder Lebensgewohnheiten - erreicht und eine Gefährdung des Patienten durch ein Beharren des Schrittmachers bei unphysiologisch hohen Raten verhindert.

Bei einem Übergang von spontaner auf stimulierte Herztätigkeit (bei Belastungserhöhung) oder umgekehrt wird zweckmäßigerweise die jeweils aktuelle Impedanzkurve als neue Referenzkurve definiert. Zur Vermeidung sprunghafter Ratenänderungen sollte zu diesem Zeitpunkt jedoch die Rate nicht oder nicht wesentlich verändert werden, was die Einführung eines Ratenoffsetbetrages erfordert, der dann über eine vorbestimmte Zeit oder Anzahl von Herzzyklen allmählich wieder verringert wird. Der Realisierung dieser Funktion dienen - neben einer entsprechenden Ausbildung der Schrittmacher- bzw. Ablaufsteuerung - eine Steuerverbindung zur Ausgangsstufe und ein Offset-Speicher.

Erfindungsgemäß ist der Schrittmacher mit einem - mindestens mittelbar mit einem Steuereingang der Integratorstufe und/oder einem Steuereingang der Ratenbestimmungseinrichtung verbundenen - Fühler für eine Aktivitätsgröße ausgerüstet ist, dessen Ausgangssignal mindestens eine der Grenzen des Integrationsbereiches und/oder eine Kennlinie der Ratenbestimmungseinrichtung einstellt. Als besonders einfacher und zugleich zweckmäßiger Fühler kommt ein digitaler Bewegungssensor in Betracht, der insbesondere eine Umschaltung zwischen verschiedenen programmierten Zeitbereichs- bzw. Integrationsgrenzen und/ oder eine entsprechende Umschaltung der Verarbeitungscharakteristik (Kennlinie) der Ratenbestimmungseinrichtung bewirkt.

In einer alternativen oder speziellen Ausbildung umfasst die Ratenbestimmungseinrichtung ein einen Dateneingang für die Impedanzgröße aufweisendes Differentialglied, mit dem sehr langsame Impedanzänderungen für die Schrittmachersteuerung unwirksam gemacht werden. Es wird hierdurch - mit drastisch verringertem Berechnungsaufwand und Stromverbrauch - ein ähnlicher Effekt wie mit der Mitführung der Impedanz-Ruhekurve erreicht.

Das Differentialglied weist in einer alternativen erfindungsgemäßen Lösung einen mit dem Fühler für die Aktivitätsgröße verbundenen Zeitkonstanten-Steuereingang auf, über den die Differentialzeit auf einen im Ruhezustand wesentlich kleineren Wert als bei Aktivität des Patienten eingestellt wird. Wird eine - oben bereits erwähnter - digitaler Bewegungssensor eingesetzt, erfolgt die Einstellung insbesondere als Umschaltung zwischen voreingestellten Zeitkonstanten.

Die die Abhängigkeit der Stimulationsrate von der Impedanzgröße bestimmende Kennlinie als wesentlicher Betriebsparameter der Ratenbestimmungseinrichtung ist bevorzugt nicht statisch, sondern wird kontinuierlich oder in bestimmten Zeitabständen optimiert. Das Ziel der Optimierung ist zunächst die Anpassung der Variationsbreite der Impedanzgröße an die zulässige Variationsbreite der Herzrate.

Die Variationsbreite ist zu Beginn des Betriebs nicht bekannt, sondern wird durch laufende Messung der Impedanz während des Betriebs ermittelt und nach jeder Messung optimiert. Zu Beginn des Betriebs wird ein Schätzwert für den unteren und den oberen Grenzwert der Variationsbreite als Startwert vorgegeben.

Bei der Optimierung sind nun zwei Fälle zu unterscheiden: Einerseits kann der Fall eintreten, dass ein Messwert der Impedanz die bisher ermittelte Variationsbreite nach oben oder unten überschreitet. In diesem Fall wird die Variationsbreite entsprechend erweitert und dadurch aktualisiert. Die Wachstumszeitkonstante dieses Anpassungsvorgangs liegt vorzugsweise in der Größenordnung weniger Sekunden, um eine schnelle Anpassung zu erreichen und so eine überhöhte Herzrate zu verhindern. Andererseits kann der Fall auftreten, dass die Impedanz die zuvor bestimmte Variationsbreite über einen längeren Zeitraum nicht mehr voll ausschöpft. In einer Ausführungsform der Erfindung wird deshalb in diesem Fall die Variationsbreite langsam wieder verringert. Die Zeitkonstante dieses Anpassungsvorgangs liegt dabei vorzugsweise in der Größenordnung mehrerer Wochen.

Die Kennlinie wird während der Optimierung jeweils an den aktuellen Wert der Variationsbreite der Aktivitätsgröße (Impedanz) angepaßt. So weist die Kennlinie dem unteren Grenzwert der Variationsbreite der Aktivitätsgröße die Basisrate zu und entsprechend dem oberen Grenzwert der Aktivitätsgröße die maximale Stimulationsrate. Durch eine Veränderung dieser Grenzwerte während der Optimierung ändert sich deshalb auch die Kennlinie.

In einer vorteilhaften Variante wird deshalb die errechnete adaptive Herzrate statistisch ausgewertet. Aus der statistischen Verteilung innerhalb der zulässigen Variationsbreite von der Basisrate bis zur maximalen Stimulationsrate lassen sich Informationen gewinnen für die Optimierung der Kennlinie. Es läßt sich für jeden Patienten in Abhängigkeit von seinem Belastungsprofil und anderen Faktoren eine physiologisch sinnvolle statistische Verteilung der Aktivitätsgröße in Form einer Häufigkeitsverteilungsfunktion bestimmen, indem der Verlauf der Kennlinie zwischen den Stützstellen so eingestellt wird, daß die Häufigkeitsverteilungsfunktion der adaptiven Herzrate der physiologisch sinnvollen Häufigkeitsverteilungsfunktion möglichst nahekommt.

Die Kennlinie läßt sich beispielsweise als Polynom realisieren, wobei die Kennlinie durch die Koeffizienten des Polynoms vollständig bestimmt ist:

$$HR(A) = K_0 + K_1 \cdot A + K_2 \cdot A^2 + K_3 \cdot A^3 + ...$$

Die Koeffizienten $K_1$ werden dann so bestimmt, daß zum einen den Grenzwerten der Variationsbreite der Aktivitätsgröße die entsprechenden Grenzwerte der zulässigen Variationsbreite der Herzrate zugeordnet werden und zum anderen die Häufigkeitsverteilungsfunktion der adaptiven Herzrate der physiologisch sinnvollen Häufigkeitsverteilungsfunktion möglichst nahekommt.

Es muß also gelten:

$$HR(A_{MIN}) = TR$$

$$HR(A_{MAX}) = MSR$$

$$V(HR) = V_{REF}$$

mit

$A_{MIN}$, $A_{MAX}$ Grenzwerte der Variationsbreite der Aktivitätsgröße

$TR$ T̲arget R̲ate (Basisrate)

$MSR$ m̲aximale S̲timulationsr̲ate

$V$ Häufigkeitsverteilungsfunktion der Herzrate

$V_{REF}$ physiologisch sinnvolle Häufigkeitsverteilungsfunktion

Zur Berechnung der Häufigkeitsverteilungsfunktion der adaptiven Herzrate wird beispielsweise die gesamte zulässige Variationsbreite in äquidistante Frequenzintervalle aufgeteilt, und für jedes Frequenzintervall die Zeit bestimmt, in der die Herzrate während eines Beobachtungszeitraums innerhalb des Frequenzintervalls lag. Um die Auswirkung zyklischer Schwankungen der adaptiven Herzrate im Tages- oder Wochenzyklus zu unterdrücken, liegt die Beobachtungsdauer hierbei vorzugsweise in der Größenordnung mehrerer Wochen.

In der Kombination von Aktivitätsfühler und Differentialglied ergibt sich insbesondere folgende Funktionsweise: Detektiert der Bewegungssensor eine Bewegung des Herzschrittmacherträgers, so werden die Differentialzeit $T_D$ und der differentiale Übertragungsfaktor $K_D$ des Differentialglieds gleichermaßen stark erhöht. Durch die Erhöhung der Differentialzeit $T_D$ wird erreicht, daß auch länger anhaltende Belastungen durch eine erhöhte Herzrate unterstützt werden. Durch die gleichzeitige Erhöhung der Differentialkonstante $K_D$ wird die Stetigkeit des Ausgangssignals des Differentialglieds sichergestellt. Andernfalls würde das Ausgangssignal des Differentialglieds bei einer Änderung der Differentialzeit $T_D$ springen, was physiologisch nicht sinnvoll ist. Nach dem Ende der physischen Belastung werden die Differentialzeit $T_D$ und der differentiale Übertragungsfaktor $K_D$ wieder auf die Werte vor der Belastungsphase zurückgesetzt.

Vorteilhafte Weiterbildungen der Erfindung sind weiterhin in den Unteransprüchen gekennzeichnet bzw. werden nachstehend zusammen mit der Beschreibung einer bevorzugten Ausführung der Erfindung anhand der Figuren näher dargestellt.

Es zeigen:

Figur 1 einen Herzschrittmacher als Ausführungsbeispiel der Erfindung als Blockschaltbild,

Figur 2 eine Kennlinie und eine optimierte Kennlinie für die Zuordnung der Aktivitätsgröße zur adaptiven Herzrate in dem in Figur 1 dargestellten Herzschrittmacher,

Figur 3 die sich aus den in Figur 2 dargestellten Kennlinien ergebenden Häufigkeitsverteilungsfunktionen der adaptiven Herzrate und

Figur 4 eine Ausführungsform einer Impedanzmeß- und -verarbeitungseinrichtung, die bei einem Herzschrittmacher ähnlich dem in Fig. 1 gezeigten eingesetzt werden kann.

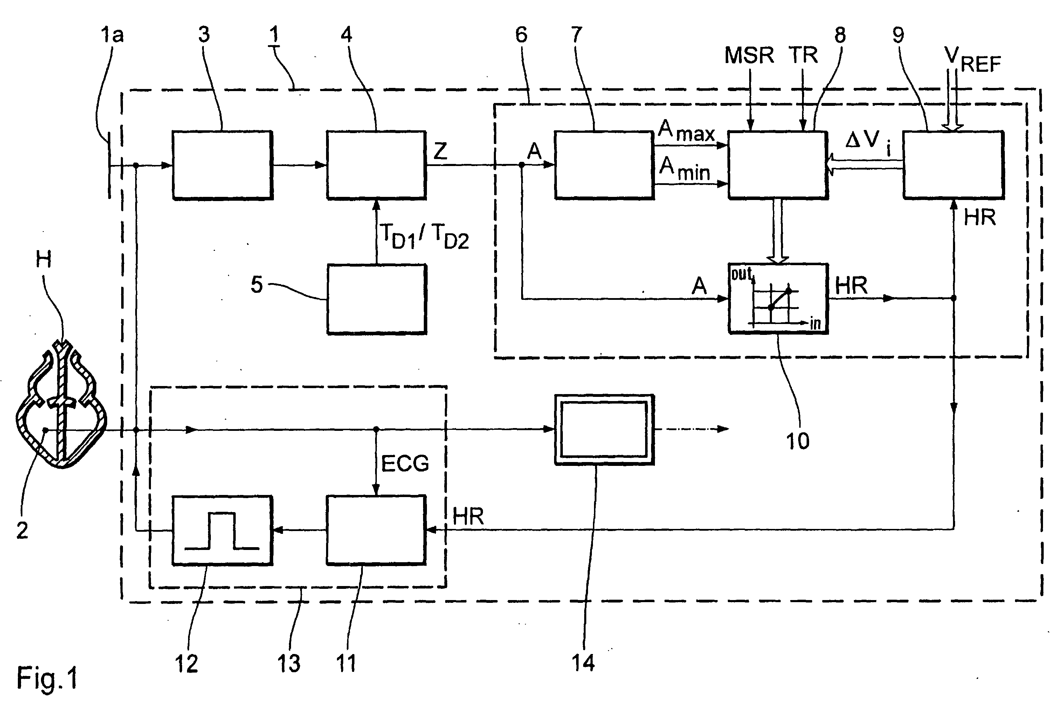

Figur 1 zeigt als Ausführungsbeispiel der Erfindung einen ratenadaptiven Herzschrittmacher 1 als Funktions-Blockschaltbild, wobei nur die für die Erläuterung der Erfindung wichtigen Komponenten dargestellt sind.

Die Impedanzmeßvorrichtung und -verarbeitungseinrichtung 3 mißt über eine unipolare Meßelektrode 2 im rechten Ventrikel des Herzens H die rechtsventrikuläre intrakardiale Impedanz Z. Die Messung erfolgt getaktet, indem die Meßelektrode 2 mit einer Meßspannung beaufschlagt und zu acht äquidistanten Zeitpunkten innerhalb eines fest vorprogrammierten Zeitbereiches der Strom zwischen der Meßelektrode 2 und dem als Gegenelektrode wirkenden Gehäuse la des Herzschrittmachers gemessen wird. Die Impedanz *Z* ergibt sich als Quotient aus Meßspannung und Strom. Die Takfrequenz der Impedanzmessung beträgt hierbei einige zehn bis ca. 100 Hz. Dadurch wird gemäß dem allgemein bekannten Abtasttheorem erreicht, daß das zeitdiskrete Impedanzsignal den tatsächlichen Verlauf der Impedanz *Z* hinreichend gut wiedergibt.

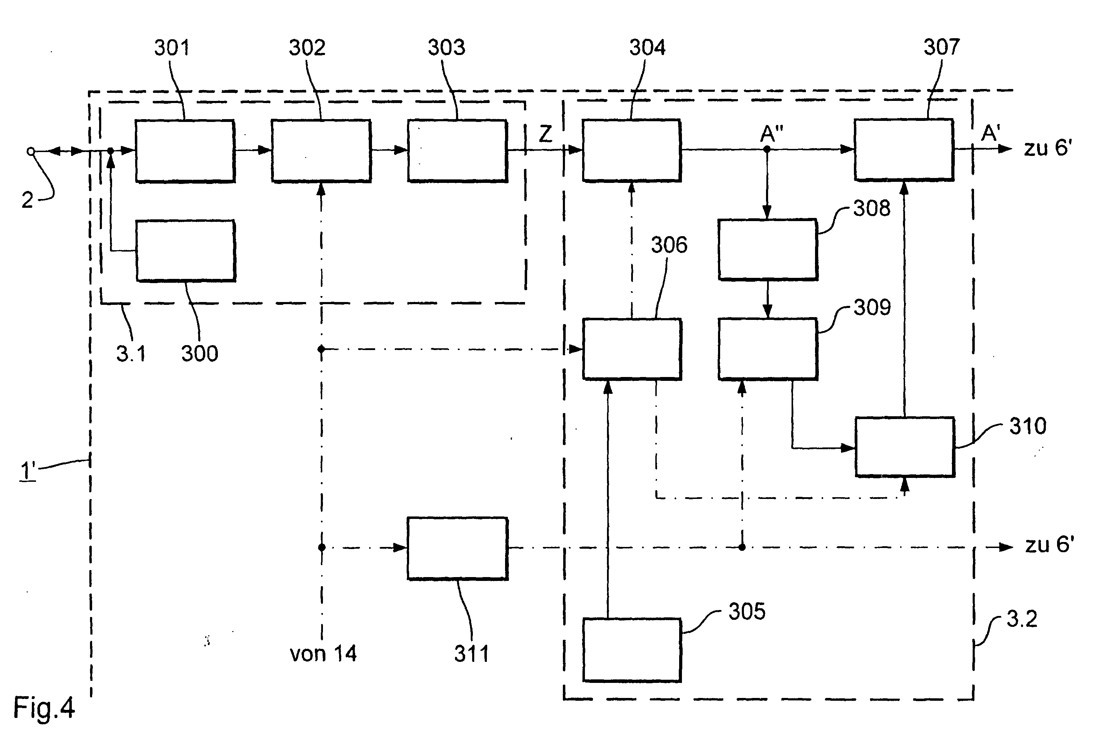

Die Impedanz *Z* weist während eines Herzzyklus relativ starke Änderungen auf. So ist die Impedanz *Z* in der Regel zu Beginn eines Herzzyklus unmittelbar nach einem QRS-Komplex minimal und steigt anschließend bis zum nächsten QRS-Komplex wieder an; ihr zeitlicher Verlauf ergibt - wie weiter unten unter Bezugnahme auf Fig. 4 genauer erläutert wird - nach Integration und arithmetischer Verarbeitung den Rateneinstellparameter.

Der Meßvorrichtung und Verarbeitungseinrichtung 3 nachgeschaltet ist ein Differentialglied 4 mit einer Differentialzeit $T_D$. Dieses Differentialglied 4 hat die Aufgabe, langsame Änderungen der intrakardialen Impedanz *Z*, deren Zeitkonstante über der Differentialzeit $T_D$ liegt, auszufiltern.

Neben der Impedanzmeßvorrichtung ist ein Bewegungssensor 5 vorgesehen, der in Abhängigkeit vom Bewegungszustand des Herzschrittmacherträgers ein binäres Signal (Bewegung ja/nein) liefert. In Abhängigkeit von diesem Bewegungssignal wird die Differentialzeit $T_D$ des Differentialglieds 4 eingestellt. Im Ruhezustand des Herzschrittmacherträgers wird eine Differentialzeit $T_{D1}$ = 10 min eingestellt. Das bedeutet, daß Änderungen der Impedanz *Z*, deren Zeitkonstante größer ist als 10 min, nicht zu einer relevanten Änderung der adaptiven Herzrate führen. Detektiert der Bewegungssensor eine Bewegung des Herzschrittmacherträgers, die mit einer physischen Belastung assoziiert ist, so wird die Differentialzeit auf $T_{D2}$ = 5 h eingestellt.

Das Ausgangssignal des Differentialglieds 4 wird einer Ratenbestimmungseinrichtung 6 zugeführt, die an ihrem Ausgang die adaptive Herzrate *HR* ausgibt.

Die Ratenbestimmungseinrichtung 6 weist ein Kennlinienglied 10 auf, das jedem Wert der - im Differentialglied nachbearbeiteten - Impedanzgröße A durch eine Kennlinie einen Wert der adaptiven Herzrate HR zuordnet. Die Kennlinie wird während des Betriebs des Herzschrittmachers laufend durch eine Stellvorrichtung 8 eingestellt und optimiert, indem die Verarbeitung der Impedanzgröße an die zulässige Variationsbreite der Herzrate *HR* angepaßt wird, die durch eine Basisrate *TR* als unterem Grenzwert und eine maximale Stimulationsrate *MSR* als oberem Grenzwert vorgegeben ist.

Die Variationsbreite der Aktivitätsgröße *A* wird durch eine (nicht gezeigte) Diskriminatoreinheit bestimmt, die laufend das Maximum $A_{MAX}$ und das Minimum $A_{MIN}$ der Impedanzgröße während der letzten vier Wochen bestimmt.

Die Kennlinie *K* ist in dem Kennlinienglied 10 als Polygonzug mit insgesamt zwölf äquidistanten Stützstellen realisiert. Eine Stützstelle ist durch den unteren Grenzwert $A_{MIN}$ des Variationsbereiches der verarbeiteten Impedanzgröße und die Basisrate *TR* und eine zweite Stützstelle durch den oberen Grenzwert $A_{MAX}$ des Variationsbereiches der Impedanzgröße und die maximale Stimulationsrate *MSR* gegeben, wobei die weiter oben genannten Beziehungen gelten. Die Lage der restlichen (im Beispiel zehn) Stützstellen läßt sich aus einem weiteren Optimierungsziel ableiten, das darin besteht, die Häufigkeitsverteilungsfunktion *V* der adaptiven Herzrate *HR* möglichst gut an eine Referenzkurve $V_{REF}$ anzupassen.

Es wird deshalb von einer Auswerteeinheit 9 die adaptive Herzrate *HR* statistisch ausgewertet. Hierzu wird laufend die Häufigkeitsverteilungsfunktion *V* der adaptiven Herzrate HR bestimmt. Dies geschieht dadurch, daß für jedes der zwischen den zwölf Stützstellen der Kennlinie *K* liegenden elf Frequenzintervalle $\Delta HR_i$ jeweils der prozentuale Zeitanteil innerhalb eines Beobachtungszeitraums bestimmt wird, für den die adaptive Herzrate *HR* innerhalb dieses Frequenzintervalls $\Delta HR_i$ lag. Die ermittelte Häufigkeitsverteilungsfunktion *V* wird mit einer Referenzkurve $V_{REF}$ verglichen, die eine physiologisch sinnvolle Häufigkeitsverteilungsfunktion der adaptiven Herzrate *HR* darstellt. Hierzu wird jeweils in den Stützstellen $V_1$ die Differenz $\Delta V_1$ zwischen der gemessenen Häufigkeitsverteilungsfunktion *V* und der Referenzkurve $V_{REF}$ gebildet und der Stellvorrichtung 8 zugeführt.

Stimmen die gemessene Häufigkeitsverteilungsfunktion *V* und die Referenzkurve $V_{REF}$ überein, so ist die Kennlinie optimal angepaßt. Andernfalls wird die Kennlinie *K* von der Stellvorrichtung 8 optimiert. Liegt in dem Frequenzintervall zwischen der ersten und der zweiten Stützstelle der Kennlinie die Häufigkeitsverteilungsfunktion *V* der Herzrate über der Referenzkurve $V_{REF}$, so bedeutet dies, daß innerhalb dieses Frequenzintervalls liegende Herzraten zu häufig auftreten. Die Steigung $^{dHR}/_{dA}$ der Kennlinie ist deshalb in diesem Frequenzintervall anzuheben. Hierzu wird die zweite Stützstelle gegen die erste Stützstelle in Richtung fallender Aktivitätswerte verschoben. Liegt dagegen die Häufigkeitsverteilungsfunktion *V* unterhalb der Referenzkurve $V_{REF}$, so bedeutet dies, daß Herzraten innerhab dieses Frequenzintervalls zu selten auftreten. Die Steigung $^{dHR}/_{dA}$ der Kennlinie ist deshalb in diesem Frequenzintervall zu verringern. Hierzu wird die zweite Stützstelle gegenüber der ersten Stützstelle in Richtungsteigender Aktivitätswerte verschoben.

In der gleichen Weise wird die neue Lage der anderen Stützstellen bestimmt. Liegt in einem zwischen zwei Stützstellen liegenden Frequenzintervall die Häufigkeitsverteilungsfunktion *V* über der Referenzkurve $V_{REF}$, so wird die zu der höheren Frequenz gehörende Stützstelle in Richtung abnehmender Aktivitäts- bzw. Impedanzgrößenwerte verschoben.

Der Steuervorrichtung ist zur Stimulation des Herzens eine Treiber- bzw. Ausgangsstufe 13 nachgeschaltet. Der Herzschrittmacher 1 arbeitet nach dem Bedarfs(Demand)-Prinzip, d.h. er stimuliert das Herz nur dann, wenn innerhalb einer bestimmten Wartezeit nach einer vorangegangenen Kontraktion des Herzens keine Kontraktion des Herzens durch eine natürliche Stimulation stattfindet. Die Treiberstufe 13 weist deshalb eine Vergleichereinheit 11 auf, die über die Meßelektrode 2 das Elektrokardiogramm (ECG) am Herzen 1 abnimmt, daraus die natürliche Herzrate bestimmt und diese mit der adaptiven Herzrate vergleicht. Wird innerhalb der Wartezeit nach einer vorangegangenen Kontraktion keine natürliche Kontraktion des Herzens 1 detektiert, so steuert die Vergleichereinheit 11 einen Impulsgeber 12 an, der einen elektrischen Stimulationsimpuls auf die (gleichzeitig als Meßelektrode dienende Reizelektrode 2 gibt.

Zur Steuerung der allgemeinen Schrittmacherfunktionen sowie zur Durchführung der Impedanzmessung und -verarbeitung ist eine eine Zeitbasis umfassende Ablaufsteuerung (Controller) 14 vorgesehen, die (symbolisch mit einem einzelnen Pfeil dargestellte) Steuersignale an die Funktionskomponenten ausgibt.

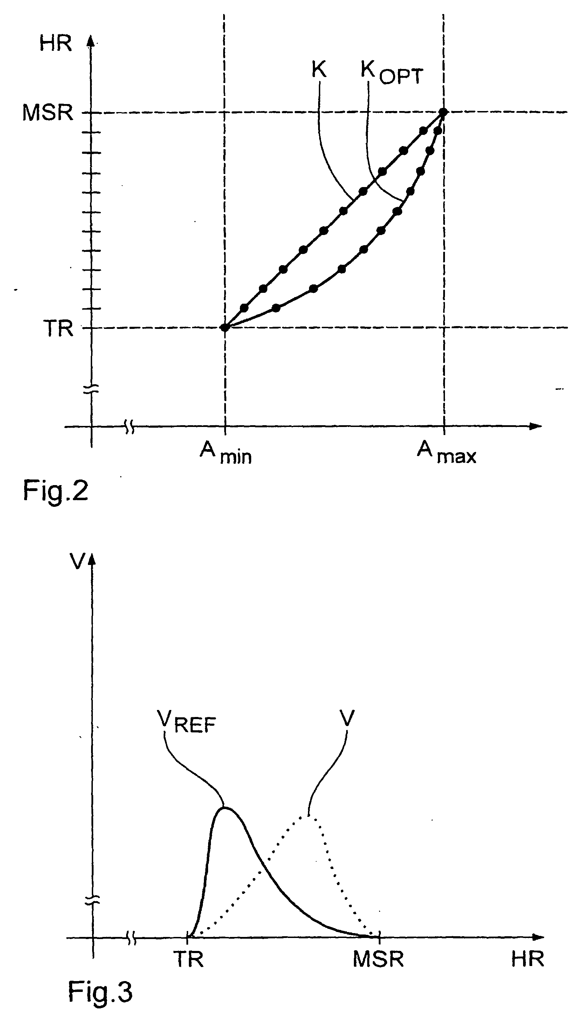

Figur 2 zeigt als ein Beispiel die Kennlinie $K$ des Kennlinienglieds 10 aus Figur 1 sowie eine optimierte Kennlinie $K_{OPT}$. Die Kennlinie $K$ wird durch einen Polygonzug mit insgesamt zwölf Stützstellen gebildet und ordnet jedem Wert der Variationsbreite der Aktivitätsgröße von $A_{MIN}$ bis $A_{MAX}$ einen Wert der adaptiven Herzrate $HR$ zu.

Die Grenzwerte der Variationsbreite der Aktivitätsgröße A sind hierbei nicht konstant. Vielmehr "lernt" der Herzschrittmacher während des Betriebs laufend, welche Variationsbreite sich bei den im täglichen Leben des Herzschrittmacherträgers auftretenden Belastungen ergibt. Die Variationsbreite der Aktivitätsgröße $A$ wird also laufend neu bestimmt. Dadurch ändert sich auch jeweils die Lage der Kennlinie $K$.

Der untere Grenzwert $A_{MIN}$ der Variationsbreite der Aktivitätsgröße $A$ und die Basisrate $TR$ bilden eine Stützstelle und der obere Grenzwert $A_{MAX}$ der Variationsbreite der Aktivitätsgröße $A$ und die maximale Stimulationsrate $MSR$ eine weitere Stützstelle der Kennlinie $K$. Zwischen diesen beiden Stützstellen verläuft die Kennlinie $K$ nach der erstmaligen Inbetriebnahme des Herzschrittmachers zunächst linear. Der Verlauf wird jedoch im Rahmen eines Optimierungsprozesses so verändert, daß die Häufigkeitsverteilungsfunktion der adaptiven Herzrate $HR$ einer physiologisch sinnvollen Referenzkurve möglichst nahekommt. Die optimierte Kennlinie $K_{OPT}$ weist im unteren Bereich der Variationsbreite der Herzrate $HR$ eine geringere Steigung $^{dHR}/_{dA}$ auf als die lineare Kennlinie $K$. Dadurch treten bei der optimierten Kennlinie $K_{OPT}$ geringere Herzraten $HR$ häufiger auf. Entsprechend ist die Steigung $^{dHR}/_{dA}$ im oberen Bereich der Variationsbreite der Herzrate $HR$ bei der optimierten Kennlinie $K_{OPT}$ größer als bei der linearen Kennlinie $K$. Hohe adaptive Herzraten $HR$ nahe der maximalen Stimulationsrate $MSR$ treten deshalb bei der optimierten Kennlinie $K_{OPT}$ seltener auf.

Figur 3 zeigt Häufigkeitsverteilungsfunktionen der adaptiven Herzrate $HR$, die sich aus den in Figur 2 dargestellten Kennlinien $K$ und $K_{OPT}$ ergeben können. Die Häufigkeitsverteilungsfunktion $V$ stellt die sich aus der Kennlinie $K$ ergebende Verteilung der adaptiven Herzrate $HR$ über die gesamte Variationsbreite der Herzrate $HR$ von der Basisrate $TR$ bis zur maximalen Stimulationsrate $MSR$ dar. Das Maximum der Häufigkeitsverteilungsfunktion $V$ liegt im oberen Frequenzbereich nahe der maximalen Stimulationsrate $MSR$, d.h. das Herz schlägt relativ oft im oberen Frequenzbereich, was physiologisch nicht sinnvoll ist. Die physiologisch sinnvolle Häufigkeitsverteilungsfunktion der adaptiven Herzrate ist durch die Referenzkurve $V_{REF}$ gegeben. Hierbei liegt das Maximum im unteren Frequenzbereich nahe der Basisrate $TR$. Die Kennlinie $K$ des Kennlinienglieds wird nach dem in Figur 2 skizzierten Prinzip so eingestellt, daß die Häufigkeitsverteilungsfunktion $V$ die Form der Referenzkurve $V_{REF}$ annimmt.

Eine zweckmäßige Ausführung der wesentlichen Komponenten zur Impedanzmessung und -verarbeitung eines gegenüber Fig. 1 geringfügig modifizierten Schrittmachers 1' zeigt Fig. 4 in Form eines Funktions-Blockschaltbildes. In Anlehnung an die in Fig. 2 gewählte Bezugsziffer 3 sind die eigentliche Impedanzmeßvorrichtung mit 3.1 und die Impedanzverarbeitungseinrichtung mit 3.2 bezeichnet.

Die eingangsseitig mit der intrakardialen Meß-, Abfühl- und Reizelektrode 2 verbundene Impedanzmeßvorrichtung 3.1 umfaßt in an sich bekanntem Aufbau einen Abtastimpulsgenerator 300, eine Filterstufe 301 zur Abtrennung störender (etwa aus der Atemtätigkeit herrührender) Signalanteile, einen Stromstärkemesser 302 und eine Impedanzberechnungsstufe 303. Die Impedanzmessungen werden durch die Ablaufsteuerung 14 (vgl. Fig. 1) in Synchronität zu stimulierten oder spontanen Herzereignissen getaktet.

Das Meßsignal Z' gelangt vom Ausgang der Impedanzberechnungsstufe 303 zum Eingang einer Integratorstufe 304, in der eine fest programmierte Anzahl (von beispielsweise acht) Impedanzmeßwerten aus einem Herzzyklus einer Integration unterzogen wird. Die Anzahl ist in einem Integrationsgrenzenspeicher 305 fest gespeichert und bestimmt den Zählwert eines die Meßsteuerimpulse von der Ablaufsteuerung zählenden Zählers 306, welcher bei Erreichen der prgrammierten Anzahl den Integrator stoppt. Der Zähler 306 löst zugleich die Übergabe des Integrationsergebnisses A'' an eine Subtraktionsstufe 307 einerseits und über ein Verzögerungsglied 308 an einen FIFO-Speicher 309 andererseits aus.

Im Speicher 309 ist ständig eine vorbestimmte Anzahl von Impedanz-Integralwerten aus der Vergangenheit (beispielsweise den jeweils letzten drei Betriebsminuten des Schrittmachers) gespeichert, die jeweils durch das Ausgangssignal des Zählers 306 in eine Mittelwertbildungsstufe 310 übernommen und einer aktuellen Mittelung unterzogen werden.

Das Ausgangssignal der Mittelwertbildungsstufe 310 wird (neben dem aktuellen Impedanz-Integralwert als primäre Impedanzgröße A") als Referenzwert A"$_{ref}$ der Subtraktionsstufe 307 zugeführt, die die Differenz zwischen dem aktuellen Integralwert und dem aktuellen Zeitmittel der Integralwerte als Referenzwert bildet und als sekundäre Impedanzgröße A' ausgibt, die hier den Ratensteuerparameter darstellt.

Zu beachten ist, daß die in Fig. 1 gezeigte Signalverbindung der Ablaufsteuerung 14 zur Meßelektrode 2, mit der auch die Herzaktionen bzw. intrakardialen EKGs erfaßt werden, eine Unterscheidung zwischen spontanen und evozierten Herzaktionen und damit ein Löschen des FIFO 309 bei einem Wechsel des Ereignistyps ermöglicht, wozu ein eingangsseitig mit der Ablaufsteuerung 14 und ausgangsseitig mit einem Löscheingang des FIFO verbundener Flipflop 311 vorgesehen ist. Dessen Ausgangssignal wird auch einer modifizierten Ratenbestimmungseinrichtung 6' zugeführt, wo der errechneten Stimulationsrate bei jedem Ereignistypwechsel ein Ratenoffset hinzugefügt wird, dessen Betrag in Abhängigkeit vom vorhergehenden und vom aktuellen Ratenwert so gewählt ist, daß der Ratensprung einen vorbestimmten Betrag nicht überschreitet, und der bei den nachfolgenden Herzereignissen stufenweise bis auf Null zurückgeführt wird. Die konkreten schaltungstechnischen Mittel zur Realisierung dieser Zusatzfunktion stehen dem Fachmann aus bekannten Anordnungen zur Ratenglättung bzw. -angleichung zur Verfügung.

Die weitere Verarbeitung der sekundären Impedanzgröße A' entspricht im übrigen - bis auf den Fortfall des durch die Komponenten zur Bildung des gleitenden Mittelwertes ersetzten - Differentialgliedes der zu Fig. 1 gegebenen Erläuterung.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch macht.

Insbesondere kann die Stufe 3.2 eine Vielzahl alternativer Ausführungen aufweisen, bei denen beispielsweise die Bildung eines gleitenden Mittelwertes als Referenzwert durch eine Zeitmittelung mit festen Startpunkten in vorbestimmtne Zeitabständen ersetzt sein oder statt anhand der Ausgangssignale der Integratorstufe 304 anhand der Ausgangssignale der Impedanzberechnungstufe 303, d.h. von (Impedanz,Zeit) -Wertepaaren, durchgeführt werden kann. Statt einer fest vorgegeben Anzahl von zu integrierenden Impedanzwerten kann auch ein fester zeitlicher Integrationsbereich programmiert sein. Es ist weiterhin in vorteilhafter Weise möglich, Mittel zur Einstellung des jeweils gültigen Integrationsbereiches in Abhängigkeit vom Signal des Bewegungssensors (oder eines anderen Aktivitätsfühlers) vorzusehen.

**Patentansprüche**

1. Ratenadaptiver Herzschrittmacher (1; 1') mit

einer Impedanzmessvorrichtung (3; 3.1) zur Messung des zeitlichen Verlaufes der, insbesondere rechtsventrikulären, intrakardialen Impedanz (Z) über mindestens einen vorbestimmten Abschnitt eines Herzzyklus,

einer Impedanzverarbeitungseinrichtung (3.2; 4) zur Gewinnung einer Impedanzgröße aus dem zeitlichen Verlauf und

einer der Impedanzverarbeitungseinrichtung nachgeschalteten und durch eine Ablaufsteuerung (14) gesteuerte Ratenbestimmungseinrichtung (6; 6') zur Bestimmung der adaptiven Stimulationsrate (HR) unter Nutzung der Impedanzgröße, wobei

die Impedanzverarbeitungseinrichtung eine Integratorstufe (304) zur Bestimmung des Zeitintegrals der Impedanz über den vorbestimmten Abschnitt des Herzzyklus als primäre Impedanzgröße (A; A") aufweist, **dadurch gekennzeichnet, dass**

ein mindestens mittelbar mit einem Steuereingang der Integratorstufe und/oder einem Steuereingang der Ratenbestimmungseinrichtung (6) verbundener Fühler (5) für eine Aktivitätsgröße vorgesehen ist, dessen Ausgangssignal mindestens eine der Grenzen des Integrationsbereiches und/oder ein Kennlinienglied (10) der Ratenbestimmungseinrichtung einstellt oder

dass die Ratenbestimmungseinrichtung ein einen Dateneingang für die Impedanzwert (Z) aufweisendes Differentialglied (4) umfasst, welches einen mit dem Fühler (5) für die Aktivitätsgröße verbundenen Zeitkonstanten-Steuereingang aufweist, wobei als Fühler für die Aktivitätsgröße ein digitaler Bewegungssensor (5) mit einem Ausgang vorgesehen ist, welcher im Ruhezustand des Patienten einen ersten Zustand und bei Bewegung einen zweiten Zustand einnimmt, und

das Differentialglied mit dem Bewegungssensor verbunden ist und dessen Ausgang die Differentialzeit (T$_D$) auf einen im Ruhezustand wesentlich kleineren Wert als bei Bewegung einstellt.

2. Aktivitätsgesteuerter Herzschrittmacher nach Anspruch 1, **dadurch gekennzeichnet, dass**

der Ausgang der Integratorstufe (304) mit einem Integralwertspeicher (309) verbunden ist, in den jeweils ein in mindestens einem vorhergehenden Herzzyklus ermittelter Referenz-Integralwert ($A''_{ref}$)gespeichert wird,

die Impedanzverarbeitungseinrichtung eine mit dem Ausgang der Integratorstufe und dem Integralwertspeicher verbundene Arithmetikeinheit (307) zur Berechnung einer sekundären Impedanzgröße (A') aus der jeweiligen primären Impedanzgröße und dem Referenz-Integralwert gemäß einer vorgegebenen arithmetischen Beziehung aufweist und

die Ratenbestimmungseinrichtung (6') zur Berechnung der adaptiven Stimulationsrate aufgrund der sekundären Impedanzgröße ausgebildet ist.

3. Ratenadaptiver Herzschrittmacher nach Anspruch 2, **dadurch gekennzeichnet, dass** die Arithmetikeinheit eine Subtraktionsstufe (307) zur Bildung des Differenzwertes zwischen der primären Impedanzgröße und dem Referenz-Integralwert aufweist.

4. Ratenadaptiver Herzschrittmacher nach einem der vorhergehenden Ansprüche, **gekennzeichnet durch** einen mindestens mittelbar mit einem Steuereingang der Integratorstufe verbundenen Festwertspeicher (305) zur Speicherung der Grenzen des vorbestimmten Abschnitts des Herzzyklus oder der Lage von Impedanzerfassungspunkten innerhalb dessen.

5. Ratenadaptiver Herzschrittmacher nach einem der Ansprüche 2 bis 4, **dadurch gekennzeichnet, dass** eine eingangsseitig mit dem Integralwertspeicher (309) und ausgangsseitig mit der Arithmetikeinheit (307) verbundene Mittelwertbildungsstufe (310) zur Bildung eines, insbesondere gleitenden, Mittelwertes aus Vergangenheits-Impedanzmessungen als Referenz-Integralwert vorgesehen ist.

6. Ratenadaptiver Herzschrittmacher nach einem der vorangehenden Ansprüche, **dadurch gekennzeichnet, dass** eine unipolare Ventrikelelelektrode (2), die insbesondere zugleich als Abfühl- und Reizelektrode geschaltet ist, mit dem Eingang der Messvorrichtung (3) verbunden ist.

**Claims**

1. Rate-adaptive pacemaker (1; 1) comprising

an impedance measuring device (3; 3.1) for measuring over time the intracardial impedance (Z), in particular right ventricular intracardial impedance, over at least one predetermined portion of a cardiac cycle;

an impedance processing device (3.2; 4) for obtaining an impedance variable from the temporal course, and

a rate determining device (6; 6'), arranged downstream of the impedance processing device and controlled by a sequence controller (14), for determining the adaptive stimulation rate (HR) using the impedance variable,

the impedance processing device comprising an integrator stage (304) for determining the time integral of the impedance over the predetermined portion of the cardiac cycle as a primary impedance variable (A; A''), **characterised in that**

a feeler (5) for an activity variable, connected at least indirectly to a control input of the integrator stage and/or a control input of the rate determining device (6), is provided, the output signal of which sets at least one of the limits of the integration range and/or a characteristic line member (10) of the rate determining device, or **in that**

the rate determining device comprises a differential member (4) having a data input for the impedance value (Z), which member comprises a time-constant control input connected to the feeler (5) for the activity variable, a digital motion sensor (5) with an output being provided as the feeler for the activity variable, which output assumes a first state when a patient is resting and a second state when the patient is in motion, and **in that**

the differential member is connected to the motion sensor and the output thereof sets the differential time ($T_D$) to a substantially lower value when the patient is resting than when the patient is in motion.

2. Activity-controlled pacemaker according to claim 1, **characterised in that**

the output of the integrator stage (304) is connected to an integral value memory (309), in which a respective reference integral value ($A''_{ref}$), which is determined in at least one preceding cardiac cycle, is stored,

the impedance processing device comprises an arithmetic unit (307), which is connected to the output of the integrator stage and the integral value memory, for calculating a secondary impedance variable (A') from the respective primary impedance variable and the reference integral value according to a predetermined arithmetical equation, and

the rate determining device (6') is configured to calculate the adaptive stimulation rate on the basis of the secondary impedance variable.

3. Rate-adaptive pacemaker according to claim 2, **characterised in that** the arithmetic unit comprises a subtraction stage (307) for forming the differential value between the primary impedance variable and the reference integral value.

4. Rate-adaptive pacemaker according to any of the preceding claims, **characterised by** a read-only memory (305), which is connected at least indirectly to a control input of the integrator stage, for storing the limits of the predetermined portion of the cardiac cycle or the location of impedance detection points therewithin.

5. Rate-adaptive pacemaker according to any of claims 2 to 4, **characterised in that** a mean value forming stage (310), which is connected on the input side to the integral value memory (309) and on the output side to the arithmetic unit (307), is provided for forming a mean value, in particular a sliding mean value, from past impedance measurements as a reference integral value.

6. Rate-adaptive pacemaker according to any of the preceding claims, **characterised in that** a unipolar ventricle electrode (2), which, in particular, is switched as both a sensing and a stimulating electrode, is connected to the input of the measuring device (3).

**Revendications**

1. Stimulateur cardiaque à rythme adaptable (1; 1'), comprenant

   un dispositif de mesure d'impédance (3;3.1) pour mesurer la variation dans le temps de l'impédance intracardiaque (Z), notamment l'impédance du ventricule droit, sur au moins une section prédéterminée d'un cycle cardiaque,
   un dispositif de traitement d'impédance (3.2;4) pour obtenir une grandeur d'impédance à partir de la variation dans le temps, et
   un dispositif de détermination de rythme (6; 6') branché en aval du dispositif de traitement d'impédance et commandé par une unité de commande d'exécution (14), pour déterminer le rythme adaptatif de stimulation (HR) moyennant l'utilisation de la grandeur d'impédance, le dispositif de traitement d'impédance comportant un étage intégrateur (304) pour déterminer l'intégrale dans le temps de l'impédance pendant la section prédéterminée du filtre cardiaque en tant que grandeur d'impédance primaire (A;A''), **caractérisé en ce que**
il est prévu un capteur (5) pour une grandeur d'activité, qui est relié au moins indirectement à une entrée de commande de l'étage intégrateur et/ou à une entrée de commande du dispositif de détermination de rythme (6) et dont le signal de sortie règle au moins l'une des limites de la zone d'intégration et/ou un circuit (10) fournissant des courbes caractéristiques du dispositif de détermination de rythme, ou que le dispositif de détermination de rythme comprend un circuit différentiel (4), qui possède une entrée de données pour la valeur d'impédance (Z) et qui possède une entrée de commande à constante de temps, qui est reliée au capteur (5) pour la grandeur d'activité, auquel cas il est prévu comme capteur pour la grandeur d'activité, un capteur de déplacement numérique (5) comportant une sortie qui, lorsque le patient est à l'état de repos, prend un premier état et dans le cas d'un déplacement prend un second état, et
que le circuit différentiel est relié au capteur de déplacement et que sa sortie règle l'intervalle de temps différentiel ($T_D$) sur une valeur nettement plus faible dans l'état de repos que dans le cas d'un déplacement.

2. Stimulateur cardiaque, dont l'activité est commandée, selon la revendication 1, **caractérisé en ce que**

   la sortie de l'étage intégrateur (304) est reliée à une mémoire de valeur intégrale (309), dans laquelle est mémorisée respectivement une valeur intégrale de référence ($A''_{ref}$) déterminée dans au moins un cycle cardiaque précédent, le dispositif de traitement d'impédance comporte une unité arithmétique (307) qui est reliée à la sortie de l'étage intégrateur et à une mémoire de valeur intégrale et sert à calculer une grandeur d'impédance secondaire (A') à partir de la grandeur d'impédance primaire respective et de la valeur intégrale de référence conformément à une relation arithmétique prédéterminée, et

   le dispositif de détermination de rythme (6') servant à calculer le rythme de stimulation adaptatif est conçu sur la base de la grandeur d'impédance secondaire.

3. Stimulateur cardiaque à rythme adaptable selon la revendication 2, **caractérisé en ce que** l'unité arithmétique comporte un étage de soustraction (307) pour former la valeur de différence entre la grandeur d'impédance primaire et la valeur intégrale de référence.

4. Stimulateur cardiaque à rythme adaptable selon l'une des revendications précédentes, **caractérisé par** une mémoire morte (305), qui est reliée au moins indirectement à une entrée de commande de l'étage intégrateur et sert à mémoriser les limites de la section prédéterminée du cycle cardiaque ou de la position des points de détection d'impédance à l'intérieur de ce dernier.

5. Stimulateur cardiaque à rythme adaptable selon l'une des revendications 2 à 4, **caractérisé en ce qu'**un étage (310) de formation de la valeur moyenne, relié côté entrée à la mémoire de valeur intégrale (309) et côté sortie à l'unité arithmétique (307), est prévu pour la formation d'une valeur moyenne, notamment d'une moyenne glissante, à partir de mesures d'impédance antérieures, en tant que valeur intégrale de référence.

6. Stimulateur cardiaque à rythme adaptable selon l'une des revendications précédentes, **caractérisé en ce qu'**une électrode ventriculaire unipolaire (2), qui est branchée notamment simultanément en tant qu'électrode de détection et de stimulation, est reliée à l'entrée du dispositif de mesure (3).

Fig.1

EP 0 793 976 B1

Fig.2

Fig.3

Fig.4

EP 0 793 976 B1

13